# FASCICULE DE BREVET EUROPEEN

(11) **EP 2 828 659 B1**
(45) Date de publication et mention de la délivrance du brevet: **02.08.2017**
(21) Numéro de dépôt: 13715343.3
(22) Date de dépôt: 18.03.2013
(51) Int. Cl.: G01N 33/542, G01N 33/50, G01N 21/64, G01N 33/53

(54) **PROCEDE DE DETERMINATION DE LA CAPACITE D'UN ANTICORPS A MAINTENIR DES CELLULES A PROXIMITE L'UNE DE L'AUTRE**
VERFAHREN ZUR BESTIMMUNG DER FÄHIGKEIT EINES ANTIKÖRPERS ZUR AUFRECHTERHALTUNG DER NÄHE VON ZELLEN ZUEINANDER
METHOD FOR DETERMINING THE ABILITY OF AN ANTIBODY TO KEEP CELLS CLOSE TO ONE ANOTHER

(30) Priorité: 19.03.2012 FR 1252433
(43) Date de publication de la demande: 28.01.2015
(73) Titulaire: Cisbio Bioassays, 30200 Codolet (FR)
(72) Inventeur: JAGA, Delphine, 30150 Sauveterre (FR)
(74) Mandataire: Marro, Nicolas
(86) Numéro de dépôt international: PCT/FR2013/050567
(87) Numéro de publication internationale: WO 2013/140074

(56) Documents cités:
- EP-A1- 2 169 404
- N. GABORIT ET AL: "Time-resolved Fluorescence Resonance Energy Transfer (TR-FRET) to Analyze the Disruption of EGFR/HER2 Dimers: A NEW METHOD TO EVALUATE THE EFFICIENCY OF TARGETED THERAPY USING MONOCLONAL ANTIBODIES", JOURNAL OF BIOLOGICAL CHEMISTRY, vol. 286, no. 13, 1 avril 2011 (2011-04-01), pages 11337-11345, XP055021021, ISSN: 0021-9258, DOI: 10.1074/jbc.M111.223503
- JIANG XU-RONG ET AL: "Advances in the assessment and control of the effector functions of therapeutic antibodies", NATURE REVIEWS DRUG DISCOVERY, vol. 10, no. 2, février 2011 (2011-02), pages 101-110, XP002684452,
- Larson et al.,: "Automatic cellular screening and characterization of therapeutic antibodies for ADCC utility.", , 2 février 2012 (2012-02-02), XP002684454, Extrait de l'Internet: URL:http://www.biotek.com/resources/articl es/cellular-screening-therapeutic-antibodi es.html [extrait le 2012-10-02]
- D. Jaga et al.,: "A New Cellular Binding Platform to measure Fc gamma receptor(FcyR) / hIgG interaction based on the Tag-lite® technology", Cisbio.com , 30 septembre 2011 (2011-09-30), XP002684453, Extrait de l'Internet: URL:http://www.htrf.com/new-cellular-bindi ng-platform-measure-fc-gamma-receptor-fc%C E%B3r-higg-interaction-based-tag-lite [extrait le 2012-10-01]

## Description

### Domaine de l'invention

L'invention est relative à un procédé de détermination de la capacité d'un anticorps candidat à maintenir des cellules à proximité l'une de l'autre, et en particulier à maintenir à proximité des cellules effectrices ou mimant des cellules effectrices portant un premier antigène ou un récepteur des fragments cristallisables Fc (ci-après « récepteurs Fc » ou FcR), avec des cellules portant un second antigène cible. L'invention est particulièrement utile pour évaluer le caractère cytotoxique d'un anticorps potentiellement utilisable comme anticorps thérapeutique.

### Etat de la technique

Les anticorps monoclonaux ont permis depuis quelques années une révolution thérapeutique. En plus des anticorps classiques, les progrès de la biologie moléculaire et de l'immunologie ont permis le développement d'anticorps possédant des domaines variables ou des paratopes différents et donc capables de reconnaître différents épitopes. Bien que l'efficacité clinique des anticorps ne soit plus à prouver, leur mode d'action chez les patients reste relativement mal connu.

L'effet thérapeutique des anticorps ciblant des antigènes membranaires passe notamment par le recrutement de cellules effectrices exprimant soit des récepteurs pour le fragment cristallisable des anticorps (ci-après, « récepteurs Fc »), soit dans le cas des anticorps recombinants bi- ou trispécifiques, par le recrutement de cellules exprimant d'autres antigènes tels que par exemple les récepteurs CD3, ou CD28. Ce dernier type d'anticorps présente l'avantage de permettre le recrutement de cellules effectrices ne possédant pas de récepteurs Fc.

Les récepteurs Fc sont des protéines présentes à la surface de certaines cellules contribuant aux fonctions du système immunitaire, en particulier des Lymphocytes NK (« natural killer », tueuses naturelles), des macrophages, des neutrophiles et des mastocytes. Leur nom provient de leur capacité à se lier à la région Fc (fragment cristallisable) des anticorps. Il en existe plusieurs types, qui sont classés en fonction du type d'anticorps qu'ils reconnaissent : les récepteurs Fc gamma (FcyR) se lient aux IgG, le récepteur Fc alpha (FcaR) se lie aux IgA et les récepteurs Fc epsilon (FcεR) se lient aux IgE.

La liaison du récepteur Fc avec un anticorps déclenche différents mécanismes en fonction de la nature de la cellule sur laquelle est exprimé ce récepteur. La table 1 est un résumé de la distribution cellulaire des différents récepteurs Fc et des mécanismes déclenchés par la liaison du récepteur à un anticorps.

**Table 1**

| **Récepteur** | **Anticorps reconnu** | **Distribution Cellulaire** | **Effet suivant liaison à l'anticorps** |
|---|---|---|---|
| **FcγRI (CD64)** | IgG1 et IgG3 | Macrophages Neutrophiles Eosinophiles Cellules dendritiques | phagocytose activation cellulaire génération d'oxygène réactif destruction de microbes |
| **FcγRIIA (CD32)** | IgG | Macrophages Neutrophiles Eosinophiles Plaquettes Cellules de Langerhans | phagocytose dégranulation (éosinophiles) |
| **FcγRIIB1 (CD32)** | IgG | Lymphocytes B Mastocytes | aucun phagocytose inhibition de l'activité des cellules |
| **FcγRIIB2 (CD32)** | IgG | Macrophages Neutrophiles Eosinophiles | phagocytose inhibition de l'activité des cellules |
| **FcγRIIIA (CD16A)** | IgG | Lymphocytes NK Macrophages (certains tissus) | induction de la cytotoxicité à médiation cellulaire dépendante d'anticorps-(ADCC) induction de la libération de cytokines par les macrophages |
| **FcγRIIIB (CD16b)** | IgG | Eosinophiles Macrophages Neutrophiles Mastocytes Cellules dendritiques folliculaires | destruction de microbes |
| **FcεRI** | IgE | Mastocytes Eosinophiles Basophiles Cellules de Langerhans | dégranulation |
| **FcεRII (CD23)** | IgE | Lymphocytes B Eosinophiles Cellules de Langerhans | molécule d'adhésion possible |
| **FcαRI (CD89)** | IgA | Monocytes Macrophages Neutrophiles Eosinophiles | phagocytose destruction de microbes |
| **Fcα / µR** | IgA et IgM | Lymphocytes B Cellules mésangiales Macrophages | endocytose destruction de microbes |

Des anticorps thérapeutiques déclenchant le mécanisme ADCC (cytotoxicité à médiation cellulaire dépendante d'anticorps) sont actuellement commercialisés (Herceptin®, Cetuximab®) et indiqués dans le traitement de certains cancers : le fragment Fc de ces anticorps se lie à un récepteur Fc présent sur les Lymphocytes NK, alors que les paratopes de ces anticorps reconnaissent un antigène présent sur des cellules tumorales (Herceptin : Her2, Cetuximab : Her1). La liaison de ces anticorps à la fois à une cellule NK et à une cellule cancéreuse entraîne notamment la destruction de cette dernière par l'activation du mécanisme ADCC.

D'autres anticorps thérapeutiques dont les paratopes sont capables de reconnaître différents antigènes, et donc capables de recruter une cellule effectrice indépendamment du recrutement médié par leur domaine Fc, ont également été développés. On peut citer entres autres, blinatumobab capable de se lier à la fois à CD19 et CD3, Catumaxomab, qui se lie à EpCAM et CD3, Hertumaxomab, qui reconnaît Her3 et CD3, MDX-210 qui se lie à Her2 et CD64, MDX-447 qui est spécifique de EGFR et CD64 ou encore rM28 qui reconnaît NG2 et CD28.

Il serait particulièrement avantageux de pouvoir détecter à la fois la liaison de l'anticorps au récepteur Fc présent sur la cellule effectrice et la liaison à son antigène cible, en particulier dans le cadre du développement d'anticorps à vocation diagnostique ou thérapeutique. De la même façon pour les anticorps bi- ou trispécifiques, il serait très utile de pouvoir détecter la liaison simultanée de l'anticorps avec ses deux différents antigènes.

Les techniques actuellement disponibles pour étudier la liaison d'anticorps aux récepteurs Fc sont relativement fastidieuses :
Le système commercialisé sous le nom Biacore® permet la détection d'interactions entre deux partenaires de liaison et est basé sur le phénomène de la résonnance plasmon de surface. Ce système nécessite l'immobilisation du récepteur Fc ou bien d'un fragment du domaine Fc de l'anticorps testé à une micropuce, capable de générer un signal qui dépend de l'indice de réfraction à sa surface (Biacore Journal Number 1, 2009, 15-17). Cette approche présente plusieurs inconvénients, notamment une étape critique de fixation d'un des partenaires de liaison à la surface de la micropuce, la nécessité d'un équipement coûteux qui requiert une certaine expertise, et le fait que la technique ne permette pas de travailler avec des cellules vivantes exprimant le récepteur Fc étudié. Par ailleurs, cette approche ne fournit pas non plus d'information quant à l'éventuelle liaison de l'anticorps testé à son antigène dans sa conformation cellulaire.

La demande de brevet Français FR2844521 décrit une approche selon laquelle un anticorps est mis en contact avec des cellules exprimant le récepteur CD16 en présence de l'antigène dudit anticorps, et au moins une cytokine produite par les cellules est mesurée, une augmentation de la quantité de cytokine étant représentative de l'activation desdites cellules. Cette technique ne permet pas la mesure directe de la liaison de l'anticorps au FcR, ni ne fournit d'information quant à la liaison dudit anticorps à son antigène. Elle nécessite également une étape de centrifugation du surnageant pour détecter les cytokines sécrétées par les cellules.

Plusieurs méthodes de laboratoire existent pour étudier l'efficacité d'anticorps ayant potentiellement un effet thérapeutique via une cellule effectrice cytotoxique, en particulier un mécanisme ADCC. Ces méthodes consistent en général à mettre en contact les anticorps étudiés avec des cellules effectrices et des cellules cibles, et à détecter la destruction des cellules cibles en mesurant :
- la radioactivité libérée par les cellules cibles (lorsqu'elles ont par exemple été chargées préalablement avec du chrome-51),
- l'activité d'enzymes de type réductases / déshydrogénases libérées dans le milieu extracellulaire et qui vont modifier les propriétés spectroscopiques de composés ajoutés dans ce milieu (tels que XTT, MTS, WST, MTT, resazurin),
- l'activité de l'enzyme GAPDH libérée dans le milieu extracellulaire, qui provoque la production d'ATP, et une émission lumineuse si l'on a préalablement introduit de la luciférase dans ce milieu (brevet US6811990),
- la libération d'Europium (lorsque les cellules cibles ont été préalablement chargées par ce composé, DELFI A® EuTDA Cytotoxicity ADOI 16 kit (PerkinElmer)),
- la coloration des cellules mortes par des produits de type bleu de tryptan, iodure de propidium.
Ces techniques présentent plusieurs inconvénients, notamment en terme de praticité de mise en oeuvre, de sensibilité, de spécificité (bruit de fond important) mais le plus significatif est qu'elles ne fournissent qu'une information indirecte et ne permettent pas de quantifier directement et de manière relativement simple la capacité d'un anticorps candidat à maintenir deux cellules à proximité l'une de l'autre.

La société Cisbio Bioassays commercialise une gamme de produit sous la dénomination Tag-lite®, permettant le marquage de protéines exprimées par des cellules par des composés fluorescents, ainsi que des composés fluorescents partenaires de FRET (HTRF®). Le phénomène de FRET entre un composé donneur d'énergie et un composé accepteur étant dépendant de la distance entre ces deux composés, les produits de la gamme Tag-lite® permettent d'étudier des interactions moléculaires dans un contexte cellulaire. Une des applications de cette technique est l'étude de l'interaction entre un récepteur couplé aux protéines G (RCPG) marqué par un partenaire de FRET, avec son ligand marqué par un deuxième partenaire de FRET. Cette approche permet par exemple d'effectuer des dosages par compétition pour évaluer la liaison de nouveaux ligands potentiels de ces RCPG. Un autre exemple d'application de ces produits est l'étude de la dimérisation de RCPG marqués par des composés partenaires de FRET.

Bacsô et al (Immunol Lett. 1996 Dec;54(2-3):151-6) ont déterminé l'efficacité d'un FRET intercellulaire entre d'une part le complexe protéique membranaire CD8 d'un lymphocyte T cytotoxique (CTL), marqué par un anticorps anti-CD8alpha conjugué avec du FITC, et d'autre part avec des antigènes MHC-I présents sur une cellule cible, marqués par un anticorps spécifique des domaines alpha 2/alpha 3 du HLA A, B, C conjugué à du TRITC. De la même façon, ces auteurs ont également déterminé l'efficacité du transfert d'énergie entre d'une part un anticorps anti-ICAMI conjugué au FITC et se fixant sur des cellules cibles, et d'autre part un anticorps anti-LFA-I conjugué au TRITC et se liant au CTL. La technique utilisée pour mesurer l'évaluation du FRET consiste à mesurer le photoblanchiement du composé donneur (FITC) et nécessite un microscope. Les auteurs ont observé un FRET au niveau des points de contact entre les cellules dans le premier cas (CD8/MHCI) mais pas dans le second (ICAMI/LFAI), ce qui selon eux confirme les connaissances de l'époque quant à l'organisation de ces molécules dans la membrane.

Larson et al. (« Automatic cellular screening and characterization of therapeutic antibodies for ADCC utility », http://www.biotek.com/resources/articles/cellular-screening-therapeutic-antibodies.html) décrit deux dosages différents : le premier ("taret receptor binding assay") vise à mesurer la liaison d'un anticorps à son antigène cible exprimé sur une cellule, et le deuxième ("CD16a cellular") permet de mesurer la liaison de la partie Fc de l'anticorps avec un récepteur CD16 exprimé sur une cellule.

Il n'existe pas aujourd'hui de méthode d'étude d'anticorps potentiellement thérapeutiques permettant de fournir en une expérience des informations quantifiables sur la capacité de ces anticorps à simultanément reconnaître leur cellule cible et à recruter une cellule effectrice, et cela dans des conditions relativement proches de celles dans lesquelles l'anticorps candidat agira *in vivo.*

Les inventeurs ont mis au point une méthode permettant de résoudre ce problème.

### BREVE DESCRIPTION DES FIGURES

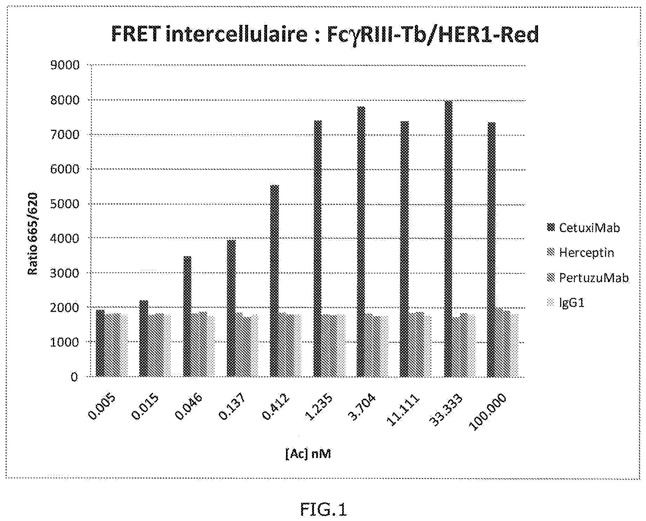

La figure 1 représente le signal de FRET émis après la mise en contact de cellules exprimant le récepteur CD16a d'une part et de cellules exprimant le récepteur HER1 d'autre part, en présence de différents anticorps, les récepteurs CD16a et HER1 étant respectivement marqués directement par un cryptate de terbium et un fluorophore accepteur rouge.

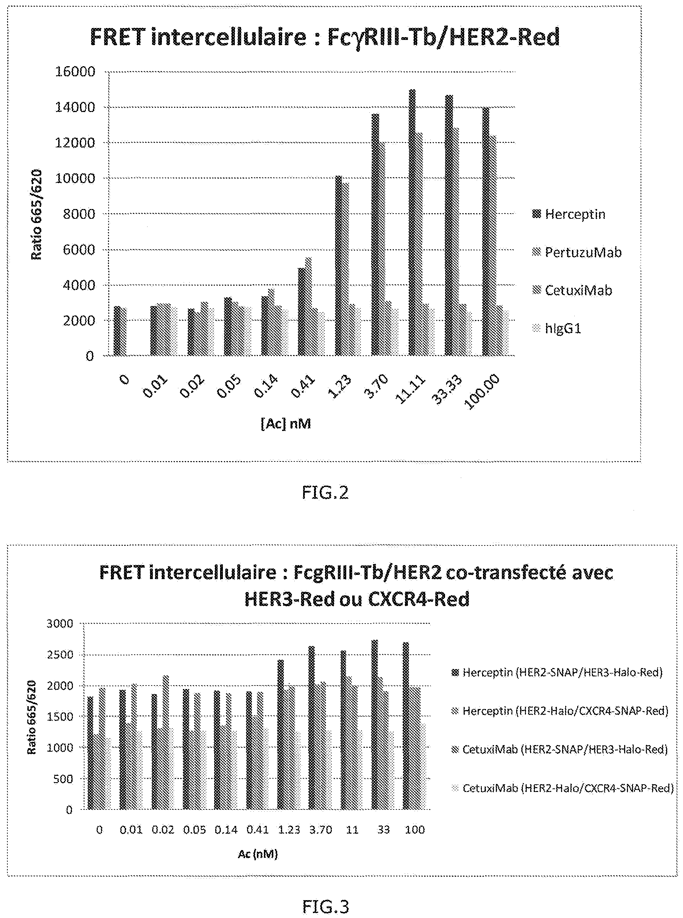

La figure 2 représente le signal de FRET émis après la mise en contact de cellules exprimant le récepteur CD16a d'une part et de cellules exprimant le récepteur HER2 d'autre part, en présence de différents anticorps, les récepteurs CD16a et HER1 étant respectivement marqués directement par un cryptate de terbium et un fluorophore accepteur rouge.
La figure 3 représente le signal de FRET émis après la mise en contact de cellules exprimant le récepteur CD16a d'une part et de cellules exprimant le récepteur HER1 d'autre part, en présence de différents anticorps, le récepteur CD16a étant marqué directement par un cryptate de terbium, et le récepteur HER1 étant marqué soit indirectement via la formation de dimères avec le récepteur HER3 lui-même marqué directement par un fluorophore accepteur rouge, ou bien par marquage non spécifique du récepteur CXCR4, qui ne forme pas de dimère avec HER1 mais dont la surexpression et la distribution homogène à la surface cellulaire permettent néanmoins l'observation d'un signal.

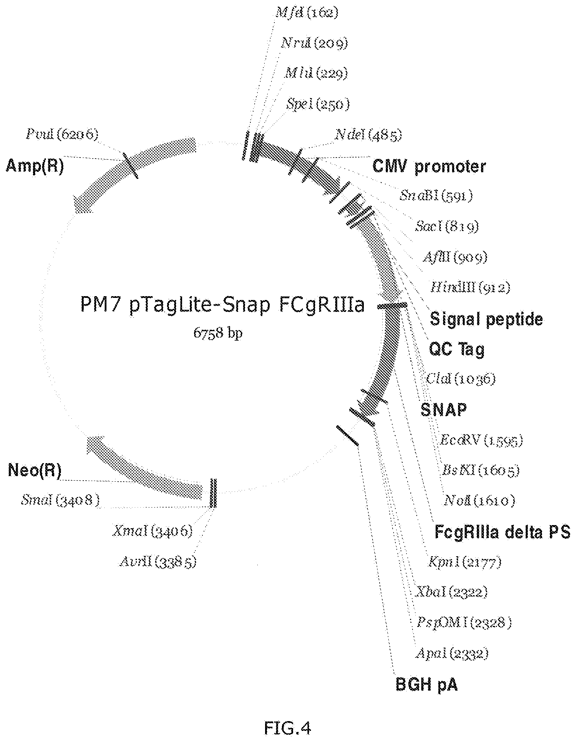

La figure 4 représente le plasmide pM7 pTagLite-Snap FcgRIIIa.

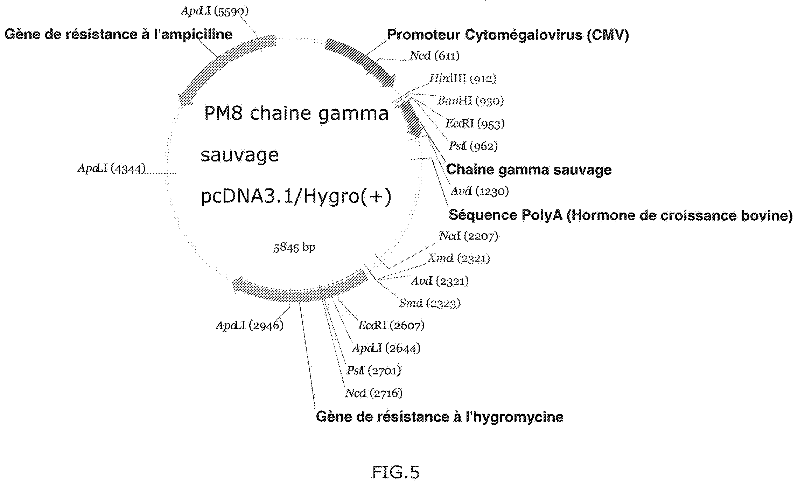

La figure 5 représente le plasmide pM8 chaîne gamma sauvage pcDNA3.1/Hygro(+).

### DESCRIPTION

L'invention est basée sur la découverte inattendue qu'il est possible de mesurer un signal de FRET entre deux cellules maintenues à proximité l'une de l'autre par un anticorps. Cette découverte est particulièrement utile pour l'étude d'anticorps potentiellement utilisables comme anticorps thérapeutiques (anticorps candidats) dont le mode d'action implique le recrutement ou rapprochement de plusieurs cellules dont une cellule effectrice, puisqu'elle permet d'étudier simplement et de quantifier au cours d'une seule expérience la liaison de l'anticorps à la fois avec sa cellule cible et avec la cellule effectrice, ou bien une cellule mimant une cellule effectrice.

Par *« anticorps candidat »,* on entend un anticorps qui a été sélectionné pour sa capacité à se lier à un antigène cible donné exprimé par exemple par une cellule cible et qui est par ailleurs capable de se lier à au moins une autre protéine, notamment mais pas seulement par l'intermédiaire de son fragment Fc ou bien via un de ses paratopes. Le terme anticorps doit être ici compris dans son sens le plus large, et inclus en plus des anticorps classiques, les composés constitués en partie d'au moins un fragment d'anticorps F(ab), d'au moins un F(ab'), d'au moins un fragment variable simple-chaîne (scFv), d'au moins un anticorps simple-domaine (sdAb) et les différentes combinaisons de ces éléments.

L'invention concerne ainsi un procédé de détermination de la capacité d'un anticorps candidat à maintenir une première cellule et une deuxième cellule à proximité l'une de l'autre, ce procédé comprenant les étapes suivantes :
- mise en contact des éléments suivants :
   (i) une première cellule exprimant dans la partie extracellulaire de sa membrane plasmique une première protéine dont on sait ou dont on suspecte qu'elle est reconnue par l'anticorps candidat, cette première protéine étant marquée de manière directe ou indirecte par le premier membre d'un couple de partenaires de FRET,
   (ii) une deuxième cellule exprimant dans la partie extracellulaire de la membrane plasmique une deuxième protéine dont on sait ou dont on suspecte qu'elle est également reconnue par l'anticorps candidat, cette deuxième protéine étant marquée de manière directe ou indirecte par le second membre dudit couple de partenaires de FRET,
   (iii) ledit anticorps candidat ;
- mesure du signal de FRET et comparaison avec le signal mesuré en l'absence d'anticorps candidat, une augmentation du signal mesuré en présence d'anticorps candidat par rapport à celui mesuré en son absence étant indicative de la capacité dudit anticorps à maintenir à proximité l'une de l'autre la première et la deuxième cellule.

Cette application inédite du principe de FRET est également particulièrement inattendue dans la mesure où l'on pouvait craindre que la distance entre les deux cellules soit trop grande pour permettre l'observation d'une variation du signal de FRET lors de la formation du complexe comprenant l'anticorps et les deux cellules.

Ce procédé permet par ailleurs d'obtenir une valeur quantifiable représentative de la liaison de l'anticorps candidat à deux protéines, et permet donc de comparer deux anticorps candidats donnés : à concentration égale, l'anticorps conduisant au signal de FRET le plus élevé aura une meilleure capacité à maintenir les deux cellules à proximité l'une de l'autre, ce qui pourrait se traduire par un effet thérapeutique amélioré.

L'expression *« couple de partenaires de FRET »* désigne un couple constitué d'un composé fluorescent donneur d'énergie (ci-après « composé fluorescent donneur ») et d'un composé accepteur d'énergie (ci-après « composé accepteur »); lorsqu'ils sont à proximité l'un de l'autre et lorsqu'ils sont excités à la longueur d'onde d'excitation du composé fluorescent donneur, ces composés émettent un signal de FRET. Il est connu que pour que deux composés fluorescents soient partenaires de FRET, le spectre d'émission du composé fluorescent donneur doit recouvrir partiellement le spectre d'excitation du composé accepteur.

Par « *signal de FRET »* on entend tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent.

L'expression « *protéine reconnue par un anticorps* » est à prendre ici au sens le plus large, c'est-à-dire que soit la protéine porte un épitope reconnu par au moins un des paratopes de l'anticorps, soit la protéine comporte un domaine capable de se lier au fragment cristallisable (fragment Fc) de l'anticorps

Dans une mise en oeuvre particulière la première cellule exprime dans la partie extracellulaire de sa membrane plasmique une première protéine qui est un récepteur membranaire du fragment Fc des anticorps, marqué de manière directe ou indirecte par le premier membre d'un couple de partenaires de FRET, et la deuxième cellule exprime dans la partie extracellulaire de sa membrane plasmique une deuxième protéine qui porte un épitope reconnu par au moins un des paratopes de l'anticorps candidat, cette deuxième protéine étant marquée de manière directe ou indirecte par le second membre dudit couple de partenaires de FRET. Cette première mise en oeuvre est particulièrement préférée car la cellule exprimant le récepteur Fc va de fait « mimer » une cellule effectrice et ainsi permettre d'obtenir de précieuses informations quant à la capacité de l'anticorps candidat à se lier à ce récepteur et par extension à recruter une cellule effectrice.

Dans cette mise en oeuvre, le récepteur Fc est choisi parmi les récepteurs de la table 1, et de manière préférée est un récepteur Fc gamma, et de manière encore plus préférée le récepteur CD16a (FcyRIIIa) ou un de ses variants. Plusieurs variants de ce récepteur sont connus, en particulier les variants naturels L66H, L66R, G147D, Y158H, F203S, F176V (ou F158V dans certaines publications). Dans une mise en oeuvre préférée, c'est le variant V158 (ou V176) qui est utilisé. Dans une autre mise en oeuvre c'est le variant F158 (ou F176).

Dans une autre mise en oeuvre particulière, l'anticorps candidat comporte deux paratopes différents, le premier paratope étant spécifique d'un premier épitope et le deuxième paratope étant spécifique d'un deuxième épitope, la première cellule exprime dans la partie extracellulaire de sa membrane plasmique une première protéine qui porte ledit premier épitope, cette première protéine étant marquée de manière directe ou indirecte par le premier membre dudit couple de partenaires de FRET, et la deuxième cellule exprime dans la partie extracellulaire de sa membrane plasmique une deuxième protéine qui porte ledit deuxième épitope, cette deuxième protéine étant marquée de manière directe ou indirecte par le second membre dudit couple de partenaires de FRET.

Dans cette mise en oeuvre, il est particulièrement avantageux que la première protéine soit une protéine présente à la surface de cellules effectrices qui ne sont pas recrutées via des récepteurs Fc, en particulier la première protéine est de préférence choisie parmi les protéines suivantes : CD3, CD28.

Le procédé selon l'invention permet des applications tout à fait intéressantes, puisqu'il constitue une nouvelle approche qui peut être intégrée aux études visant à déterminer si un anticorps donné peut potentiellement provoquer une réponse cytotoxique médiée par une cellule effectrice.

Ainsi, l'invention concerne également un procédé de détermination du caractère cytotoxique d'un anticorps candidat, ce procédé consistant à mettre en oeuvre le procédé décrit ci-dessus avec une première cellule mimant une cellule effectrice. Selon ce procédé, l'apparition d'un signal de FRET en présence de l'anticorps candidat est représentative des affinités de cet anticorps pour la cellule cible et la cellule effectrice, qui sont généralement corrélées à son efficacité thérapeutique.

Par cellule effectrice, on entend une cellule capable de provoquer directement ou indirectement, la mort d'une autre cellule, et selon l'invention, une cellule « mimant une cellule effectrice » est une cellule exprimant à sa surface une ou plusieurs protéines exprimées normalement par de telles cellules effectrices, mais n'ayant pas leur effet cytotoxique.

Dans cette mise en oeuvre, la première cellule exprime à sa surface une ou plusieurs protéines exprimées normalement à la surface d'une cellule effectrice choisie parmi les cellules suivantes : Lymphocyte NK, Macrophage, Lymphocyte T cytotoxique, Lymphocyte T auxiliaire (« helper »), soit une cellule exprimant une protéine choisie parmi : CD16a, CD3, CD28.

De préférence, la première cellule exprime à sa surface une ou plusieurs protéines exprimées normalement par un lymphocyte NK, à la base du mécanisme ADCC qui est le mode d'action le plus fréquent des anticorps thérapeutiques. Dans ce cas et de manière encore plus préférée, la première cellule exprime un récepteur Fc tel que décrit ci-dessus, et en particulier le récepteur CD16a.

### Marquage des protéines reconnues par l'anticorps candidat

### (a) Couplage des protéines membranaires reconnues par l'anticorps candidat avec un donneur ou un accepteur de manière indirecte (non covalente)

Une protéine membranaire peut être couplée avec un composé fluorescent membre d'un couple de partenaires de FRET de manière indirecte via une seconde protéine capable de s'associer avec elle, cette seconde protéine étant elle-même marquée de manière directe ou indirecte par un composé fluorescent. Il est par exemple connu que le récepteur FcγRIIIA (CD16a) est associé dans la membrane cellulaire à la chaîne gamma du récepteur FcεRI ou à la sous unité zeta de CD3 ; le récepteur CD16a peut ainsi être marqué de manière indirecte via ces chaînes gamma ou zeta, elles-mêmes marquées directement par un composé fluorescent. De la même façon, si l'une des protéines reconnue par l'anticorps candidat est un récepteur membranaire formant un dimère avec une autre protéine, il sera possible de marquer cette autre protéine. Cela est illustré par exemple dans l'exemple 4 dans lequel la protéine HER2 est marquée de manière indirecte via HER3 avec laquelle elle forme un dimère et qui est, elle, marquée de manière directe par un fluorophore.

De manière tout à fait surprenante, il a été découvert que le marquage d'une protéine membranaire quelconque, c'est-à-dire ne s'associant pas particulièrement à l'une des protéines reconnues par l'anticorps candidat, suffit à mettre en oeuvre le procédé selon l'invention, sous réserve que la protéine en question soit largement exprimée au niveau de la membrane plasmique. Cela peut s'expliquer par le modèle de « mosaïque fluide » que constitue la membrane cellulaire, c'est-à-dire par le fait que les protéines membranaires naviguent à la surface des cellules et qu'une protéine largement exprimée va se retrouver à un moment ou à un autre à proximité de la protéine reconnue par l'anticorps candidat et qu'un signal de FRET va pouvoir être mesuré. Cette mise en oeuvre a été illustrée dans l'exemple 4 dans lequel la protéine HER2 est coexprimée avec la protéine CXCR4 avec laquelle elle ne s'associe pas, mais qui est suffisamment surexprimée pour qu'un signal de FRET soit observé en présence de l'anticorps candidat.

Une protéine membranaire peut également être couplée avec un composé fluorescent par l'intermédiaire d'un couple de partenaires de liaison dont l'un au moins est de nature protéique. Dans cette approche, la protéine membranaire est fusionnée avec le partenaire de liaison de nature protéique par les techniques classiques de biologie moléculaire (construction d'un vecteur d'expression comprenant une séquence de nucléotides codant pour la protéine membranaire, fusionnée avec celle codant pour le partenaire de liaison protéique, et introduction du vecteur d'expression dans la cellule). Le donneur ou l'accepteur est conjugué de manière covalente à l'autre partenaire de liaison, que l'on appelle ici agent de couplage, qui sera ensuite ajouté au milieu extracellulaire. La reconnaissance des partenaires de liaison permet le marquage indirect de la protéine membranaire par le donneur ou l'accepteur.

A titre d'exemple non limitatif de ce type de partenaires de liaison, on peut citer :
- Le couple constitué par un anticorps spécifique d'un épitope naturellement présent sur la protéine membranaire, marqué par un composé fluorescent, et cet épitope. Lorsque cette méthode de marquage est utilisée, il convient de vérifier que la liaison de cet anticorps à cet épitope ne gêne pas la liaison de l'anticorps candidat à cette protéine, que ce soit via son fragment Fc ou via son paratope. Par ailleurs, dans cette mise en oeuvre, il convient d'éviter d'utiliser un anticorps dont le fragment constant pourrait être reconnu par la protéine membranaire d'intérêt.
- Le couple constitué de la séquence cystéine-cystéine-X-X-cystéine-cystéine (SEQ ID N°1) dans laquelle X est un acide aminé quelconque et d'un composé bi-arsenic. Ces composés bi-arsenic peuvent être facilement marqués avec une molécule organique du type fluorescéine ou rhodamine (voir B.A.Griffin et al. (1998) Science. 1998, 281, 269-271 et S.A. Adams et al. (2002) J. Am. Chem. Soc. 2002, 124, 6063-6076 pour des détails sur la technologie).
- Le peptide BTX (bungarotoxine), composé de 13 acides aminés qui est reconnu par la bungarotoxine (BTX), peut être couplé à une molécule fluorescente (voir C. M. McCann et al. (2005), Biotechnique (2005), 38, 945-952).
- Le couple streptavidine (ou avidine) / biotine : la séquence de liaison de la Streptavidine (SBP-Tag) est une séquence formée par 38 acides aminés qui présente une haute affinité pour la biotine pouvant être préalablement marquée avec un donneur ou un accepteur (voir C. M. McCann et al. (2005), Biotechnique (2005), 38, 945-952).
- La séquence de l'enzyme dihydrofolate réductase d'E. coli (eDHFR) qui lie spécifiquement et avec une haute affinité des ligands, tels que la triméthoprime sur lesquels peuvent être greffés le donneur ou l'accepteur selon la technologie dénommée « Ligand link Universal labelling technology » de la Société Active Motif.
- Les couples tags/antitags sont des partenaires de liaison fréquemment utilisés pour marquer des protéines. Le terme « tag » désigne une petite « étiquette » protéique constituée d'une séquence d'acides aminés, généralement mais pas obligatoirement assez courte (moins de 15 acides aminés), qui est fusionnée à la protéine membranaire que l'on souhaite marquer. Le terme « antitag » désigne un anticorps se liant de manière spécifique audit « tag ». Dans cette mise en oeuvre, l'anticorps « antitag » est lié de manière covalente au donneur ou à l'accepteur. Lorsque l'anticorps ainsi marqué est ajouté au milieu extracellulaire, il se lie au « tag » conjugué à la protéine membranaire et l'interaction « tag/antitag » permet le marquage indirect de cette protéine par le donneur ou l'accepteur. A titre d'exemple non limitatif de couples « tags/antitags », on peut citer les couples suivants dont les membres sont disponibles commercialement : GST/anticorps anti-GST dans lequel GST représente la glutathione S-transférase ou un de ses fragments ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG/anticorps anti-FLAG dans lequel FLAG est un peptide ayant les 8 acides aminés DYKDDDDK (SEQ ID N°2) ; HA/anticorps anti HA dans lequel HA est un épitope de l'hémaglutinine d'Influenza, constitué des 9 acides aminés YPYDVPFYA (SEQ ID N°3). Il est clair que la nature exacte du tag n'est pas critique pour la mise en oeuvre de l'invention.

### (b) Couplage des protéines membranaires reconnues par l'anticorps candidat avec un donneur ou un accepteur de manière directe (covalente)

Dans cette approche, le donneur ou l'accepteur est couplé à la protéine membranaire par une liaison covalente ; plusieurs techniques ont été décrites et les réactifs nécessaires à leurs mises en oeuvre sont disponibles commercialement. Pour ce couplage, on pourra utiliser l'une des techniques ci-après :
- Formation d'une liaison covalente au niveau d'un groupe réactif présent sur la protéine membranaire, en particulier au niveau d'un des groupes suivants : le groupe amino terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.
   Ces groupes présents sur la protéine membranaire peuvent former une liaison covalente avec un groupe réactif porté par le donneur ou l'accepteur. Les groupes réactifs appropriés sont connus de l'homme du métier : un donneur ou l'accepteur fonctionnalisé par un groupe maléimide sera par exemple capable de se lier de manière covalente avec les groupes thiols portés par les cystéines de la protéine. De même, un donneur/accepteur portant un ester de N-hydroxysuccinimide sera capable de se fixer de manière covalente à une amine du récepteur membranaire.
- Utilisation d'une enzyme suicide
   Par enzyme suicide on entend des protéines qui ont une activité enzymatique modifiée par des mutations spécifiques qui leur confèrent la capacité de lier rapidement et de façon covalente un substrat. Ces enzymes sont dites « suicides » car chacune ne peut lier qu'une seule molécule fluorescente, l'activité de l'enzyme étant bloquée par la fixation du substrat. Ces enzymes constituent par conséquent un outil de choix pour marquer spécifiquement des récepteurs d'intérêt avec un rapport d'une molécule fluorescente pour une protéine. Dans cette approche, une enzyme suicide est fusionnée, par les techniques classiques de biologie moléculaire, avec la protéine membranaire - de préférence dans sa partie N-terminale - et le substrat de l'enzyme lié de manière covalente à un donneur/accepteur est introduit dans le milieu extracellulaire. La réaction enzymatique a pour conséquence la liaison covalente du substrat marqué à l'enzyme, et donc le marquage de la protéine membranaire par le donneur ou l'accepteur.

On peut citer à titre d'exemple non limitatif les enzymes suivantes :
- les mutants de l'O6-alkylguanine DNA alkyltransférase (AGT). Les enzymes SNAP-tag (Juillerat et al, Chemistry & biology, Vol.10, 313-317 Avril 2003) et CLIP-tag (Gautier et al, Chemistry et Biology, 15, 128-136, février 2008) commercialisées par la société Cisbio Bioassays sont des mutants de l'AGT humaine dont les substrats sont respectivement, l'O⁶-benzylguanine (ci-après abrégée BG) et l'O²-benzylcytosine (ci-après abrégée BC). L'enzyme N-AGT (Gronemeyer et al. (Protein engineering, design & selection, vol. 19, no 7, pp 309-3016, 2006)) est un autre mutant de cette enzyme dont la réactivité avec l'O⁶-benzylguanine est meilleure que celle de l'enzyme SNAP-tag.
- les mutants d'une déshalogénase (telle que l'enzyme HaloTag commercialisée par Promega) qui génère également une réaction enzymatique du type suicide (voir WO2004/072232), dont certains des substrats sont des composés de la famille des chloroalcanes, en particulier les chloroalcanes comportant le motif -NH-CH₂CH₂-O-CH₂CH₂-O-(CH₃)₆-Cl. Dans ce cas, le donneur/accepteur sera conjugué à ce type de motif.
- La protéine ACP (« Acyl Carrier Protein »), protéine transporteur d'acyles, sur laquelle est transféré, en présence de phosphopantéthéine transférase, le résidu 4'-phosphopantéthéine du coenzyme A sur une sérine de l'ACP (N. George et al, Journal of the American Chemical society 126 (2004) p 8896-8897). Lorsque cette approche est utilisée pour marquer la protéine membranaire par le donneur ou l'accepteur, il est nécessaire de rajouter au milieu réactionnel de la phosphopantéthéine transférase. La société NEB commercialise un fragment de l'ACP sous le nom commercial « ACP-Tag » pour le marquage de protéines.

Lorsque cette approche est utilisée pour marquer une protéine membranaire d'intérêt, les cellules sont transfectées avec un plasmide d'expression comportant l'ADN codant pour une protéine de fusion comprenant l'enzyme suicide et le récepteur d'intérêt. Ce plasmide peut également comprendre, en amont de l'ADN codant pour ces protéines, l'ADN codant pour une étiquette telle que par exemple l'épitope FLAG, l'épitope myc, ou encore celui de l'hémaglutinine de l'influenza (HA). Ces étiquettes ne sont pas essentielles mais facilitent la manipulation de la protéine de fusion à des fins de contrôle ou de purification. La transfection est opérée par les techniques classiques, telle que l'électroporation.

Pour s'assurer que la protéine de fusion sera exprimée dans la membrane cellulaire, il peut être utile d'inclure dans le plasmide d'expression, en amont de la séquence codant pour le récepteur d'intérêt et de l'enzyme suicide, celle codant pour un peptide d'adressage membranaire, tels que le peptide signal T8 ou le peptide signal du récepteur mGluR5, dont l'utilisation à cet effet est connue de l'homme du métier. Enfin, il peut également être souhaitable de s'assurer que la séquence codant pour le récepteur d'intérêt ne comporte pas de séquence d'adressage membranaire native qui pourrait faire l'objet d'un clivage post-traductionnel de la liaison entre le récepteur d'intérêt et l'enzyme suicide : si c'est le cas, il est préférable de ne pas introduire ce domaine dans le plasmide d'expression.

Finalement, lorsqu'une enzyme suicide est utilisée pour marquer une protéine membranaire avec un partenaire de FRET, l'invention comprend une étape préliminaire de transfection des cellules par un vecteur d'expression comprenant la séquence d'ADN codant pour une protéine de fusion correspondant à la protéine membranaire, fusionné dans sa partie N-terminale avec une enzyme suicide. Les techniques de transfection telles que l'électroporation ou l'utilisation de lipofectamine sont connues de l'homme du métier.

L'introduction dans le milieu extracellulaire du substrat de l'enzyme conjugué à un partenaire de FRET aura pour conséquence le marquage du récepteur d'intérêt par ce partenaire de FRET.

La société Cisbio Bioassays commercialise des plasmides Tag-Lite® permettant l'expression de protéines de fusion avec les enzymes suicides connues sous les noms commerciaux de SNAP-tag®, CLIP-tag® et Halotag®. Les séquences d'ADN codant pour les récepteurs Fc de la table 1 sont connues et sont disponible dans les bases de données telles que Genbank.

Ainsi, dans un mode de réalisation de l'invention mettant en oeuvre des enzymes suicides, soit la première protéine, soit la deuxième protéine, soit la première et la deuxième protéines sont marquées de manière directe via une enzyme suicide, à savoir qu'elle est ou qu'elles sont exprimées sous forme d'une protéine de fusion comprenant la première ou la deuxième protéine et l'enzyme suicide, et le marquage est effectué par mise en contact des cellules exprimant ces protéines avec le substrat de l'enzyme, conjugué à un des partenaires de FRET.

### Vecteurs d'expression, Nécessaire de réactifs, Cellules

Les vecteurs d'expression pour la mise en oeuvre de l'invention sont des plasmides permettant l'expression d'une protéine de fusion comprenant la séquence d'ADN d'une protéine à laquelle peut se lier l'anticorps candidat (via ses paratopes ou son fragment Fc) et la séquence codant pour une enzyme suicide, en particulier choisie parmi les mutants de la déshalogénase, un fragment de la protéine de transport d'acyles ou les mutants de l'O6-alkylguanine DNA alkyltransférase, ces derniers étant préférés. Ils peuvent être obtenus en intégrant la séquence d'ADN codant pour la protéine d'intérêt dans un des plasmides commercialisé par la société Cisbio Bioassays sous les dénominations Tag-lite®, SNAP-tag®, CLIP-tag® et Halo-tag®.

Les premières et deuxièmes cellules pour la mise en oeuvre de l'invention sont des cellules, notamment des cellules de mammifères, qui ont été transfectées de manière stable ou transitoire avec les vecteurs d'expression selon l'invention. Les techniques d'introduction de vecteurs d'expression dans les cellules, telle que l'électroporation ou encore l'utilisation de lipofectamine, sont connues de l'homme du métier. Dans un aspect particulièrement avantageux, ces cellules ont été incubées en présence du substrat de l'enzyme suicide, conjugué à un membre d'un couple de partenaires de FRET, et le gène d'intérêt est ainsi marqué. Ces cellules peuvent être conditionnées sous forme congelée pour faciliter leur conservation et leur distribution aux utilisateurs.

La présente demande décrit également des nécessaires ou trousses de réactifs pour la mise en oeuvre du procédé selon l'invention. Ces nécessaires ou trousses comprennent au minimum les composants suivants, qui peuvent être conditionnés et vendus ensemble ou bien séparément :
- des cellules de mammifères exprimant une première protéine membranaire fusionnée à une enzyme suicide ;
- des cellules de mammifères exprimant une deuxième protéine membranaire fusionnée à une enzyme suicide ;
- les substrats des enzymes suicides, conjugués aux membres d'un couple de partenaires de FRET.

Ces réactifs permettent de marquer les cellules pour ensuite mettre en oeuvre le procédé selon l'invention avec un anticorps candidat capable ou susceptible de se lier à la première et à la deuxième protéine. Les enzymes suicides sont identiques ou différentes et sont choisies de préférence parmi : les mutants de la déshalogénase, un fragment de la protéine de transport d'acyles ou les mutants de l'O6-alkylguanine DNA alkyltransférase. Si les enzymes suicide fusionnées aux premières et deuxièmes protéines sont différentes, le marquage des deux cellules par les partenaires de FRET pourra être effectué dans un même milieu réactionnel. Si les enzymes suicides sont les mêmes, alors chaque population de cellule sera marquée séparément.

Un nécessaire de réactifs particulièrement avantageux consiste à fournir des cellules prémarquées et congelées, qui sont directement utilisables pour mettre en oeuvre le procédé selon l'invention avec un anticorps candidat capable ou susceptible de se lier à une première et une deuxième protéine. Un tel nécessaire comprend:
- des cellules de mammifères exprimant une première protéine membranaire fusionnée à une enzyme suicide et marquée par le premier membre d'un couple de partenaires de FRET ;
- des cellules de mammifères exprimant une deuxième protéine membranaire fusionnée à une enzyme suicide et marquée par le deuxième membre d'un couple de partenaires de FRET.

### Couples de partenaires de FRET

Selon l'invention, des protéines membranaires sont marquées par un membre d'un couple de partenaires de FRET, en particulier par un composé fluorescent donneur ou un composé fluorescent accepteur d'énergie.

Le FRET est défini comme un transfert d'énergie non radiatif résultant d'une interaction dipôle-dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera émis.

La sélection du couple de fluorophores donneur / accepteur pour obtenir un signal de FRET est à la portée de l'homme du métier. Des couples donneur-accepteur utilisables pour étudier les phénomènes de FRET sont notamment décrits dans l'ouvrage de Joseph R. Lakowicz (Principles of fluorescence spectroscopy, 2nd edition 338), auquel l'homme du métier pourra se référer.

Les composés fluorescents donneurs d'énergie à durée de vie longue (> 0,1 ms, de préférence comprise entre 0,5 et 6 ms), en particulier les chélates ou cryptates de terres rares sont avantageux puisqu'ils permettent d'effectuer des mesures en temps résolu, c'est-à-dire de mesurer des signaux de TR-FRET (en langue anglaise, « Time Resolved FRET ») en s'affranchissant d'une grande partie du bruit de fond émis par le milieu de mesure. Ils sont pour cette raison et de manière générale préférés pour la mise en oeuvre du procédé selon l'invention. Avantageusement, ces composés sont des complexes de lanthanides. Ces complexes (tels que chélates ou cryptates) sont particulièrement appropriés en tant que membre du couple de partenaires de FRET donneur d'énergie.

Les complexes de dysprosium (Dy3+), de samarium (Sm3+), de néodymium (Nd3+), d'ytterbium (Yb3+) ou encore d'erbium (Er3+) sont des complexes de terres rares également appropriés aux fins de l'invention, mais les complexes d'europium (Eu3+) et de terbium (Tb3+) sont particulièrement préférés.

De très nombreux complexes de terres rares ont été décrits et plusieurs sont actuellement commercialisés par les sociétés PerkinElmer, Invitrogen et Cisbio Bioassays.

Des exemples de chélates ou cryptates de terres rares appropriés aux fins de l'invention sont :
- Les cryptates de lanthanides, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassays. On peut citer à titre d'exemple non limitatif les cryptates d'europium de formules ci-dessous (qui peuvent être couplés au composé à marquer via un groupe réactif, ici par exemple un groupe NH₂) : 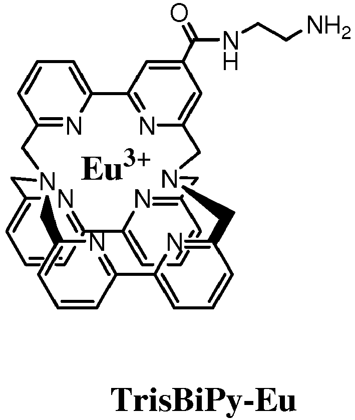 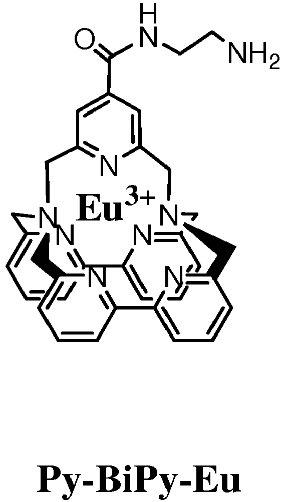 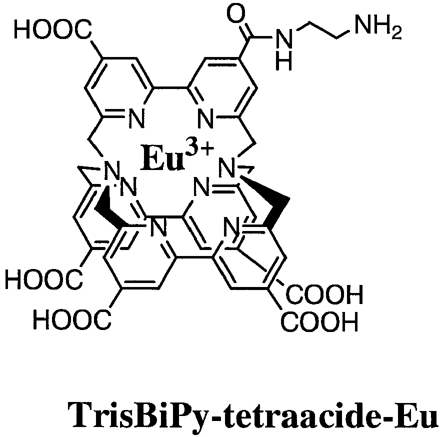 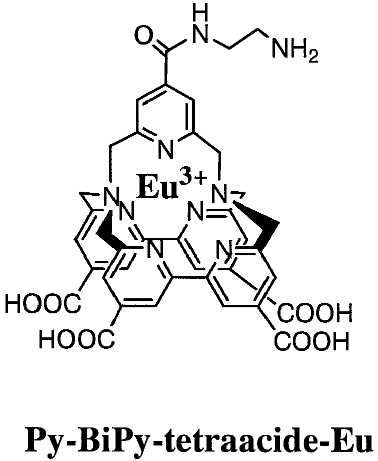
- Les chélates de lanthanides décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates comprenant un ligand nonadenté tel que la terpyridine. Des chélates de lanthanides sont commercialisés par la société PerkinElmer.
- Des complexes de lanthanides comprenant un agent chélatant, tel que le tétraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO 2009/10580.
- Les cryptates de lanthanides décrits dans les brevets EP 1 154 991 et EP 1 154 990 sont également utilisables.
- Le cryptate de terbium de formule ci-dessous (qui peut être couplé à un composé à marquer via un groupe réactif, ici par exemple un groupe NH₂) : 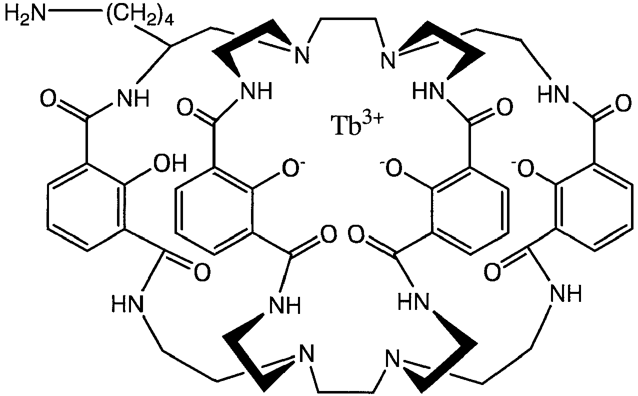 et dont la synthèse est décrite dans la demande internationale WO 2008/063721 (composé 6a page 89).
- Le cryptate de terbium Lumi4-Tb de la société Lumiphore, commercialisé par Cisbio Bioassays.
- Le quantum dye de la société Research Organics, de formule ci-dessous (qui peut être couplé au composé à marquer via un groupe réactif, ici NCS) : 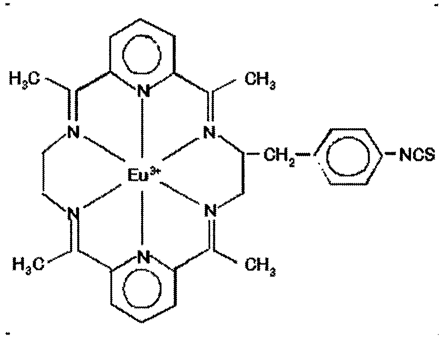
- Les chélates de ruthénium, en particulier les complexes constitués d'un ion ruthénium et de plusieurs bipyridines comme le ruthénium(II) tris(2,2'-bipyridine).
- Le chélate de terbium DTPA-cs124 Tb, commercialisé par la société Life technologies de formule ci-dessous (qui peut être couplé au composé à marquer via un groupe réactif R) et dont la synthèse est décrite dans le brevet américain US 5 622 821. 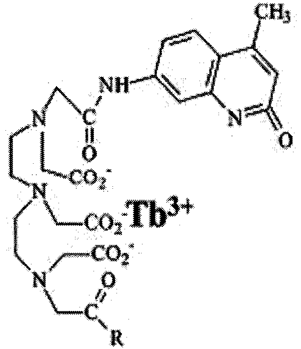
- Le chélate de terbium de formule ci-dessous et décrit par Latva et al (Journal of Luminescence 75: 149-169) :

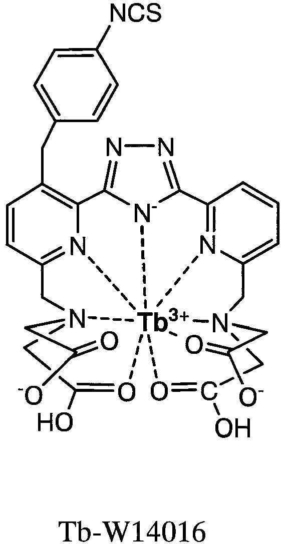

De manière particulièrement avantageuse, le composé fluorescent donneur est choisi parmi : un cryptate d'europium; un chélate d'europium ; un chélate de terbium ; un cryptate de terbium ; un chélate de ruthénium ; et un quantum dye ; les chélates et les cryptates d'europium et de terbium étant particulièrement préférés.

Les complexes de dysprosium (Dy3+), de samarium (Sm3+), de néodymium (Nd3+), d'ytterbium (Yb3+) ou encore d'erbium (Er3+) sont également des complexes de terres rares appropriés aux fins de l'invention.

Les composés fluorescents accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination «DY», les composés connus sous la dénomination « Alexa », le nitrobenzoxadiazole. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles dans le commerce.

Les composés « Alexa » sont commercialisés par la société Invitrogen; les composés « Atto » sont commercialisés par la société Attotec ; les composés « »DY» » sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Les protéines fluorescentes suivantes peuvent également être utilisées en tant que composé fluorescent accepteur : les protéines fluorescentes cyans (AmCyan1, Midori-Ishi Cyan, mTFP1), les protéines fluorescentes vertes (EGFP, AcGFP, TurboGFP, Emerald, Azami Green, ZsGreen), les protéines fluorescentes jaunes (EYFP, Topaz, Venus, mCitrine, YPet, PhiYFP, ZsYellow1, mBanana), les protéines fluorescentes oranges et rouges (Orange kusibari, mOrange, tdtomato, DsRed, DsRed2, DsRed-Express, DsRed-Monomer, mTangerine, AsRed2, mRFP1, JRed, mCherry, mStrawberry, HcRed1, mRaspberry, HcRed-Tandem, mPlim, AQ143), les protéines fluorescentes dans le rouge lointain (mKate, mKate2, tdKatushka2).

Aux fins de l'invention, les dérivés de cyanines ou de la fluorescéine sont préférés comme composés fluorescents accepteurs.

L'invention est illustrée par les exemples suivants donnés à titre purement indicatif.

### Exemple 1 : Matériel et méthode

### Réactifs utilisés

*Milieu de culture* : DMEM/Glutamax + 10 % SVF+ streptomycine (50µg/ml), pénicilline 50 U/ml, HEPES 2mM, acides aminés non essentiels 1% (InVitrogen)
OptiMEM : milieu de culture utilisé pour la transfection, Invitrogen
*Lipofectamine 2000*: Invitrogen
*Plasmide SNAP-CD16A* (pM7 pTagLite-Snap FcgRIIIa) : préparé en insérant la séquence nucléique (SEQ ID N°4) codant pour le récepteur CD16A (variant V158 (ou V176) ref. Genbank NM_000569.6, séquence protéique NP_000560.5) dans le plasmide SNAP-tag® pT8-SNAP-Neomycin (Cisbio Bioassays), voir figure 4.
*Plasmide chaîne gamma du récepteur FcεRI* (pM8 chaîne gamma sauvage pcDNA3.1/Hygro(+) : la séquence d'ADN (SEQ ID N°5) codant pour la chaine gamma du récepteur FcεRI a été insérée dans un plasmide d'expression par les techniques classiques. La carte de ce plasmide est représentée à la figure 5. Il est connu que le récepteur CD16a forme des dimères avec cette chaîne gamma, et il est donc conseillé de coexprimer les deux protéines. Les inventeurs ont néanmoins découvert que cette co-expression n'était pas nécessaire pour mettre en oeuvre le procédé selon l'invention, même si elle est recommandée.
*Plasmides Halo-HER1, Halo-HER2 et Halo-HER3:* préparés en insérant la séquence nucléique codant pour le récepteur HER1, HER2 ou HER3 (séquence protéique ref uniprot P00533, P04626, P21860) dans le plasmide pT8-HaloTag-Neomycin (Cisbio Bioassays)
*Plasmides SNAP-HER2 et SNAP-CXCR4 :* préparés en insérant la séquence nucléique codant pour le récepteur HER2 ou CXCR4 (ref. uniprot P04626 et P61073) dans le plasmide SNAP-tag® pT8-SNAP-Neomycin (Cisbio Bioassays)
*SVF :* sérum de veau fétal, Invitrogen
*DMSO :* Diméthylsulfoxide, SIGMA
*Tag-lite*® *SNAP-Lumi4Tb :* Composé donneur, Cisbio Bioassays, ref SSNPTBX
*Tag-lite*® *HALO-Red* : Composé accepteur rouge, Cisbio Bioassays ref SHALOREDE
*Tag*-*lite*® *SNAP-Red* : Composé accepteur rouge, Cisbio Bioassays ref SSNPREDE
*Tag-lite*® : tampon de marquage, Cisbio Bioassays, ref LABMED.
*Mélange de transfection CD16*/*Chaîne gamma :* Mélange constitué d'une part de 5 µl de plasmide « SNAP-CD16A » (1µg/µl), 15 µl de Lipofectamine et 2,6 ml de milieu OptiMEM et d'autre part de 10 µl de plasmide « chaîne gamma » (1µg/µl), 30 µl de Lipofectamine et 5,4 ml de milieu OptiMEM
*Mélange de transfection HER1* : Mélange constitué de 20 µl de plasmide « Halo-HER1 » (1µg/µl), 60 µl de Lipofectamine et 8 ml de milieu OptiMEM
*Mélange de transfection HER2 :* Mélange constitué de 20 µl de plasmide « Halo-HER2 » (1µg/µl), 60 µl de Lipofectamine et 8 ml de milieu OptiMEM
*Mélange de transfection HER2 et HER3 :* Mélange constitué de 10 µl de plasmide « SNAP-HER2 » (1µg/µl), 30 µl de Lipofectamine et 4 ml de milieu OptiMEM et de 10 µl de plasmide « Halo-HER3 » (1µg/µl), 30 µl de Lipofectamine et 4 ml de milieu OptiMEM
*Mélange de transfection HER2 et CXCR4* : Mélange constitué de 10 µl de plasmide « Halo-HER2 » (1µg/µl), 30 µl de Lipofectamine et 4 ml de milieu OptiMEM et de 10 µl de plasmide « SNAP-CXCR4 » (1µg/µl), 30 µl de Lipofectamine et 4 ml de milieu OptiMEM
*Cetuximab :* anticorps spécifique de HER1
*Herceptin* : anticorps spécifique de HER2
*Pertuzumab :* anticorps spécifique de HER2

### Transfection des cellules

Le mélange de transfection (CD16/Chaîne gamma , HER1, HER2 , HER2/HER3 ou HER2/CXCR4) a été incubé pendant 20 minutes à température ambiante.

Des cellules HEK293 ont été mises en culture dans une flasque T175. Une fois que ces cellules ont atteint une confluence de 60 à 70%, le milieu de culture a été retiré et les cellules ont été lavées avec 10 ml de milieu PBS. 8 ml du mélange de transfection ont ensuite été ajoutés à ces cellules, ainsi que 12 ml de milieu de culture. Les cellules ont ensuite été incubées pendant 1 nuit à 37°C.

### Marquage des cellules avec les composés fluorescents

Après retrait du mélange de transfection et lavage avec 10 ml de PBS, 10 ml de l'un des composés fluorescent suivant ont été ajoutés :
- Tag-lite® SNAP-Lumi4-Tb (composé donneur, 100nM) en solution dans le tampon de marquage Tag-lite® pour les cellules transfectées avec le mélange de transfection CD16a/chaîne gamma
- Tag-lite® HALO-Red (composé accepteur, 100nM) en solution dans le tampon de marquage Tag-lite® pour les cellules transfectées avec les mélanges de transfection HER1, HER2 et HER2/HER3.
- Tag-lite® SNAP-Red (composé accepteur, 100nM) en solution dans le tampon de marquage Tag-lite® pour les cellules transfectées avec les mélanges de transfection HER2/CXCR4.

Après incubation de 1h à 37°C, le mélange a été lavé 4 fois avec le tampon de marquage Tag-lite®, puis on a ajouté 5 ml de tampon « cell dissociation buffer » (Sigma) pour dissocier les cellules et 5 ml de milieu OptiMEM. Les cellules ainsi obtenues ont été centrifugées pendant 5min à 1200 tr/mn. Le culot a ensuite été resuspendu avec 1 ml + 1 ml de tampon de marquage Tag-lite® afin de pouvoir compter les cellules.

Cette suspension a été re-centrifugée 5 min à 1200 tr/mn et le culot a été repris par du milieu de culture contenant du SVF 10% et DMSO 10% pour obtenir une suspension de cellules marquées à la concentration de 1 million de cellules / ml.

Cette suspension a été aliquotée dans des tubes à raison de 1 ml par tube, et les tubes ont été placés dans une boite Nalgene à -80°C.

### Exemple 2 : FRET HEK-CD16a et HEK-HER1

Les cellules exprimant les protéines HEK-CD16a et HEK-HER1 préparées et marquées par des composés fluorescents dans l'exemple 1 ont été décongelées à 37°C et rapidement mélangées à 15 ml de PBS. La suspension obtenue a été centrifugée 5 min à 1200 tr/mn et le surnageant a été retiré. Le culot a été re-suspendu dans du tampon Tag-lite® pour obtenir une suspension cellulaire. Les cellules HEK-CD16a et HEK-HER1 ont été distribuées dans les puits d'une plaque multipoint 384 LV aux concentrations respectives de 10 000 cellules par puits sous 5µl et 20 000 cellules par puits sous 5µl.

Un anticorps (Cetuximab, Herceptin, Pertuzumab ou un anticorps humain quelconque d'isotype IgG1 comme contrôle négatif) a été ajouté à différentes concentrations finales de 0,01 nM à 100 nM sous 10 µl. La plaque 384 a été lue immédiatement, puis après 30 min, 1h, 2h30, 3h40, 5h, 6h et 12h.

Les résultats présentés sur la figure 1 montrent que seul l'anticorps Cetuximab, qui est connu pour se lier à la fois à CD16a via son fragment Fc et à HER1 via ses domaines variables, provoque l'apparition d'un signal de FRET. Ce signal résulte du rapprochement des cellules exprimant CD16a et de celles exprimant HER1 et montre que le procédé selon l'invention permet de déterminer la capacité d'un anticorps à de maintenir deux cellules à proximité l'une de l'autre, et cela de manière quantitative.

### Exemple 3 : FRET HEK-CD16a et HEK-HER2

La même expérience que celle de l'exemple 2 a été réalisée mais cette fois avec des cellules HEK-HER-2 à la place des cellules HEK-HER-1.

Les résultats présentés sur la figure 2 montrent là encore que le procédé selon l'invention permet de distinguer les anticorps se liant à la fois au récepteur CD16a et à HER2 (Herceptin, pertuzumab), des anticorps ne se liant qu'au récepteur CD16a (Cetuximab, Anticorps hIgG1).

### Exemple 4 : FRET HEK-CD16a et HEK-HER2-HER3 ou HEK-HER2-CXCR4, marquage indirect

La même expérience que celle de l'exemple 2 a été réalisée mais cette fois avec des cellules HEK-HER2-HER3 marquée sur HER3 ou HEK-HER2-CXCR4 marquées sur CXCR4, à la place des cellules HEK-HER-1. Cette expérience vise à montrer que le procédé selon l'invention peut être mis en oeuvre par un marquage indirect d'une des protéines reconnues par l'anticorps candidat, ici le marquage indirect de HER2 par HER3 ou CXCR4.

Les résultats présentés sur la figure 3 montrent là aussi que le procédé selon l'invention permet de distinguer les anticorps se liant à la fois au récepteur CD16a et à HER2 (Herceptin), des anticorps ne se liant qu'au récepteur CD16a (Cetuximab), même si le signal observé ici est moins élevé que dans les exemples précédents.

## Revendications

1. Procédé de détermination de la capacité d'un anticorps candidat à maintenir une première cellule et une deuxième cellule à proximité l'une de l'autre, ce procédé comprenant les étapes suivantes :
- mise en contact des éléments suivants :
(i) une première cellule exprimant dans la partie extracellulaire de sa membrane plasmique une première protéine dont on sait ou dont on suspecte qu'elle est reconnue par l'anticorps candidat, cette première protéine étant marquée de manière directe ou indirecte par le premier membre d'un couple de partenaires de FRET,
(ii) une deuxième cellule exprimant dans la partie extracellulaire de la membrane plasmique une deuxième protéine dont on sait ou dont on suspecte qu'elle est également reconnue par l'anticorps candidat, cette deuxième protéine étant marquée de manière directe ou indirecte par le second membre dudit couple de partenaires de FRET,
(iii) ledit anticorps candidat ;
- mesure du signal de FRET et comparaison avec le signal mesuré en l'absence d'anticorps candidat, une augmentation du signal mesuré en présence d'anticorps candidat par rapport à celui mesuré en son absence étant indicative de la capacité dudit anticorps à maintenir à proximité l'une de l'autre la première et la deuxième cellule.

2. Procédé selon la revendication 1, **caractérisé en ce que** ladite première protéine est un récepteur membranaire du fragment Fc des anticorps, et **en ce que** ladite deuxième protéine porte un épitope reconnu par au moins un des paratopes de l'anticorps candidat.

3. Procédé selon la revendication 2, **caractérisé en ce que** le récepteur Fc est un récepteur Fc gamma.

4. Procédé selon l'une quelconque des revendications 2 et 3, **caractérisé en ce que** le récepteur Fc est le récepteur CD16a ou un de ses variants.

5. Procédé selon la revendication 1, **caractérisé en ce que** l'anticorps candidat comporte deux paratopes différents, le premier paratope étant spécifique d'un premier épitope et le deuxième paratope étant spécifique d'un deuxième épitope, **en ce que** ladite première protéine porte ledit premier épitope, et **en ce que** ladite deuxième protéine porte ledit deuxième épitope.

6. Procédé selon la revendication 5, **caractérisé en ce que** ladite première protéine est choisi parmi : CD3, CD28.

7. Procédé de détermination du caractère cytotoxique d'un anticorps candidat, ce procédé consistant à mettre en oeuvre le procédé selon l'une quelconque des revendications 1 à 6 avec une première cellule mimant une cellule effectrice choisie parmi les cellules suivantes : Lymphocyte NK, Macrophage, Lymphocyte T cytotoxique, Lymphocyte T auxiliaire.

8. Procédé de détermination du caractère cytotoxique d'un anticorps candidat, ce procédé consistant à mettre en oeuvre le procédé selon l'une quelconque des revendications 1 à 6, avec une première cellule qui exprime une protéine choisie parmi : CD16a, CD3, CD28.

9. Procédé selon l'une quelconque des revendications 1 à 8, **caractérisé en ce que** soit la première protéine, soit la deuxième protéine, soit la première et la deuxième protéines sont marquées de manière directe via une enzyme suicide, à savoir qu'elle est ou qu'elles sont exprimées sous forme d'une protéine de fusion comprenant la première ou la deuxième protéine et l'enzyme suicide, et que le marquage est effectué par mise en contact des cellules exprimant ces protéines avec le substrat de l'enzyme, conjugué à un des partenaires de FRET.

10. Procédé selon la revendication 9, **caractérisé en ce que** l'enzyme suicide est choisie parmi : les mutants de l'O6-alkylguanine DNA alkyltransférase, les mutants de la déshalogénase, ou un fragment de la protéine de transport d'acyles.

## Patentansprüche

1. Verfahren zur Bestimmung der Kapazität eines Antikörpers, der ein Kandidat dafür ist, eine erste Zelle und eine zweite Zelle in der Nähe voneinander zu halten, wobei das Verfahren die folgenden Schritte umfasst:
- Inkontaktbringen der folgenden Elemente:
(i) einer ersten Zelle, die im extrazellulären Teil ihrer Plasmamembran ein erstes Protein exprimiert, von dem bekannt ist oder von dem vermutet wird, dass es von dem Kandidaten-Antikörper erkannt wird, wobei dieses erste Protein direkt oder indirekt von dem ersten Mitglied eines FRET-Partnerpaars markiert wird,
(ii) einer zweiten Zelle, die im extrazellulären Teil der Plasmamembran ein zweites Protein exprimiert, von dem bekannt ist oder von dem vermutet wird, dass es ebenso von dem Kandidaten-Antikörper erkannt wird, wobei dieses zweite Protein direkt oder indirekt von dem zweiten Mitglied des FRET-Partnerpaars markiert wird,
(iii) des Kandidaten-Antikörpers,
- Messen des FRET-Signals und Vergleichen mit dem in Abwesenheit des Kandidaten-Antikörpers gemessenen Signals, wobei ein Anstieg des in Anwesenheit des Kandidaten-Antikörpers gemessenen Signals in Bezug auf jenes, das in seiner Abwesenheit gemessen wird, die Kapazität des Antikörpers anzeigt, die erste und die zweite Zelle in der Nähe voneinander zu halten.

2. Verfahren nach Anspruch 1, **dadurch gekennzeichnet, dass** das erste Protein ein Membranrezeptor des Fc-Fragments von Antikörpern ist, und dadurch, dass das zweite Protein ein Epitop trägt, das von mindestens einem der Paratope des Kandidaten-Antikörpers erkannt wird.

3. Verfahren nach Anspruch 2, **dadurch gekennzeichnet, dass** der Fc-Rezeptor ein Fc gamma-Rezeptor ist.

4. Verfahren nach einem der Ansprüche 2 und 3, **dadurch gekennzeichnet, dass** der Fc-Rezeptor der CD16a-Rezeptor oder eine seiner Varianten ist.

5. Verfahren nach Anspruch 1, **dadurch gekennzeichnet, dass** der Kandidaten-Antikörper zwei verschiedene Paratope umfasst, wobei das erste Paratop für ein erstes Epitop spezifisch ist, und das zweite Paratop für ein zweites Epitop spezifisch ist, dadurch, dass das erste Protein das erste Epitop trägt, und dadurch, dass das zweite Protein das zweite Epitop trägt.

6. Verfahren nach Anspruch 5, **dadurch gekennzeichnet, dass** das erste Protein ausgewählt wird aus: CD3, CDE28.

7. Verfahren zur Bestimmung des zytotoxischen Charakters eines Kandidaten-Antikörpers, wobei dieses Verfahren darin besteht, das Verfahren nach einem der Ansprüche 1 bis 6 mit einer ersten Zelle durchzuführen, welche eine Effektorzelle nachahmt, die aus den folgenden Zellen ausgewählt wird: NK-Lymphozyt, Makrophage, zytotoxischer T-Lymphozyt, T-Helfer-Lymphozyt.

8. Verfahren zur Bestimmung des zytotoxischen Charakters eines Kandidaten-Antikörpers, wobei dieses Verfahren darin besteht, das Verfahren nach einem der Ansprüche 1 bis 6 mit einer ersten Zelle durchzuführen, die ein Protein exprimiert, das ausgewählt wird aus: CD16a, CD3, CD28.

9. Verfahren nach einem der Ansprüche 1 bis 8, **dadurch gekennzeichnet, dass** entweder das erste Protein, oder das zweite Protein, oder das erste und das zweite Protein direkt über ein Suizid-Enzym markiert wird oder werden, nämlich dass dieses oder dass diese in der Form eines Fusionsproteins, umfassend das erste oder das zweite Protein und das Suizid-Enzym, exprimiert wird oder werden, und dadurch, dass die Markierung durch Inkontaktbringen der diese Proteine exprimierenden Zellen mit dem Enzym-Substrat, konjugiert an einen der FRET-Partner, durchgeführt wird.

10. Verfahren nach Anspruch 9, **dadurch gekennzeichnet, dass** das Suizid-Enzym ausgewählt wird aus: den Mutanten von O6-Alkylguanin-DNA-alkyltransferase, den Mutanten von Dehalogenase, oder einem Fragment des Acyl-Transport-Proteins.

## Claims

1. A method for determining the ability of a candidate antibody to keep a first cell and a second cell close to one another, this method comprising the following steps:
- bringing the following elements in contact:
(i) a first cell expressing in the extracellular portion of its plasma membrane a first protein that is known to be or is suspected of being recognized by the candidate antibody, said first protein being labelled directly or indirectly with the first member of a pair of FRET partners,
(ii) a second cell expressing in the extracellular portion of the plasma membrane a second protein that is also known to be or suspected of being recognized by the candidate antibody, said second protein being labelled directly or indirectly with the second member of said pair of FRET partners,
(iii) said candidate antibody;
- measuring the FRET signal and comparison with the signal measured in the absence of the candidate antibody, wherein an increase in the signal measured in the presence of the candidate antibody relative to that measured in its absence being indicative of the ability of said antibody to keep the first cell and the second cell close to one another.

2. The method as claimed in claim 1, wherein said first protein is a membrane receptor of the Fc fragment of the antibodies, and wherein said second protein bears an epitope recognized by at least one of the paratopes of the candidate antibody.

3. The method as claimed in claim 2, wherein the Fc receptor is an Fc gamma receptor.

4. The method as claimed in any one of claims 2 to 3, wherein the Fc receptor is the CD16a receptor or a variant thereof.

5. The method as claimed in claim 1, wherein the candidate antibody comprises two different paratopes, the first paratope being specific for a first epitope and the second paratope being specific for a second epitope, wherein said first protein bears said first epitope, and wherein said second protein bears said second epitope.

6. The method as claimed in claim 5, wherein said first protein is selected from: CD3, CD28.

7. A method for determining the cytotoxic character of a candidate antibody, said method consisting of carrying out the method as claimed in any one of claims 1 to 6 with a first cell imitating an effector cell selected from the following cells: NK Lymphocyte, Macrophage, cytotoxic T Lymphocyte, Helper T cell.

8. A method for determining the cytotoxic character of a candidate antibody, said method consisting of carrying out the method as claimed in any one of claims 1 to 6, with a first cell which expresses a protein selected from: CD16a, CD3, CD28.

9. The method as claimed in any one of claims 1 to 8, wherein either the first protein, or the second protein, or the first and the second proteins are labelled directly via a suicide enzyme, namely it is or they are expressed in the form of a fusion protein comprising the first or the second protein and the suicide enzyme, and wherein the labelling is carried out by bringing the cells expressing these proteins into contact with the substrate of the enzyme, conjugated to one of the FRET partners.

10. The method as claimed in claim 9, wherein the suicide enzyme is selected from: the mutants of O6-alkylguanine DNA alkyltransferase, the mutants of dehalogenase, or a fragment of the acyl carrier protein.


### SEQUENCE LISTING

<110> CISBIO BIOASSAYS
<120> Procédé de détermination de la capacité d'un anticorps à maintenir des cellules à proximité l'une de l'autre
<130> 1H305110 0006 FR
<160> 5
<170> Patent In version 3.5
<210> 1
   <211> 6
   <212> PRT
   <213> Artificial Sequence
<220>
   <223> partenaire de liaison
<220>
   <221> misc_feature
   <222> (3)..(4)
   <223> Xaa can be any naturally occurring amino acid
<400> 1
   Cys Cys Xaa Xaa Cys Cys
   1               5
<210> 2
   <211> 8
   <212> PRT
   <213> artificial sequence
<220>
   <223> flag/anticorps anti-flag
<400> 2
   Asp Tyr Lys Asp Asp Asp Asp Lys
   1               5
<210> 3
   <211> 9
   <212> PRT
   <213> artificial sequence
<220>
   <223> HA/anticorps anti HA
<400> 3
   Tyr Pro Tyr Asp Val Pro Phe Tyr Ala
   1               5
<210> 4
   <211> 6758
   <212> DNA
   <213> artificial sequence
<220>
   <223> plasmide
<220>
   <221> promoter
   <222> (232)..(819)
   <223> promoteur CMV
<220>
   <221> six_peptide
   <222> (923)..(986)
<220>
   <221> tag
   <222> (1004)..(1027)
<220>
   <221> Snaptag
   <222> (1046)..(1591)
<220>
   <221> gene
   <222> (1610)..(2321)
   <223> gene d'intérêt
<220>
   <221> polyA_site
   <222> (2358)..(2582)
   <223> séquence polyA d'HCB
<220>
   <221> gene
   <222> (3466)..(4260)
   <223> gène de résistance à la néomycine
<220>
   <221> gene
   <222> (5762)..(6622)
   <223> gène de résistance à l'ampicilline
<400> 4
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagctttgag acatggcctt accagtgacc gccttgctcc tgccgctggc 960
   cttgctgctc cacgccgcca ggccggccgc cgctagcggc atcgactaca aggacgacga 1020
   tgacaaggcc ggcatcgatg ccatcatgga caaagactgc gaaatgaagc gcaccaccct 1080
   ggatagccct ctgggcaagc tggaactgtc tgggtgcgaa cagggcctgc acgagatcaa 1140
   gctgctgggc aaaggaacat ctgccgccga cgccgtggaa gtgcctgccc cagccgccgt 1200
   gctgggcgga ccagagccac tgatgcaggc caccgcctgg ctcaacgcct actttcacca 1260
   gcctgaggcc atcgaggagt tccctgtgcc agccctgcac cacccagtgt tccagcagga 1320
   gagctttacc cgccaggtgc tgtggaaact gctgaaagtg gtgaagttcg gagaggtcat 1380
   cagctaccag cagctggccg ccctggccgg caatcccgcc gccaccgccg ccgtgaaaac 1440
   cgccctgagc ggaaatcccg tgcccattct gatcccctgc caccgggtgg tgtctagctc 1500
   tggcgccgtg gggggctacg agggcgggct cgccgtgaaa gagtggctgc tggcccacga 1560
   gggccacaga ctgggcaagc ctgggctggg tgatatccag cacagtggcg gccgcgaaga 1620
   tctcccaaag gctgtggtgt tcctggagcc tcaatggtac agggtgctcg agaaggacag 1680
   tgtgactctg aagtgccagg gagcctactc ccctgaggac aattccacac agtggtttca 1740
   caatgagagc ctcatctcaa gccaggcctc gagctacttc attgacgctg ccacagtcga 1800
   cgacagtgga gagtacaggt gccagacaaa cctctccacc ctcagtgacc cggtgcagct 1860
   agaagtccat atcggctggc tgttgctcca ggcccctcgg tgggtgttca aggaggaaga 1920
   ccctattcac ctgaggtgtc acagctggaa gaacactgct ctgcataagg tcacatattt 1980
   acagaatggc aaaggcagga agtattttca tcataattct gacttctaca ttccaaaagc 2040
   cacactcaaa gacagcggct cctacttctg cagggggctt gtcgggagta aaaatgtgtc 2100
   ttcagagact gtgaacatca ccatcactca aggtttggca gtgtcaacca tctcatcatt 2160
   ctttccacct gggtaccaag tctctttctg cttggtgatg gtactccttt ttgcagtgga 2220
   cacaggacta tatttctctg tgaagacaaa cattcgaagc tcaacaagag actggaagga 2280
   ccataaattt aaatggagaa aggaccctca agacaaatga tctagagggc ccgtttaaac 2340
   ccgctgatca gcctcgactg tgccttctag ttgccagcca tctgttgttt gcccctcccc 2400
   cgtgccttcc ttgaccctgg aaggtgccac tcccactgtc ctttcctaat aaaatgagga 2460
   aattgcatcg cattgtctga gtaggtgtca ttctattctg gggggtgggg tggggcagga 2520
   cagcaagggg gaggattggg aagacaatag caggcatgct ggggatgcgg tgggctctat 2580
   ggcttctgag gcggaaagaa ccagctgggg ctctaggggg tatccccacg cgccctgtag 2640
   cggcgcatta agcgcggcgg gtgtggtggt tacgcgcagc gtgaccgcta cacttgccag 2700
   cgccctagcg cccgctcctt tcgctttctt cccttccttt ctcgccacgt tcgccggctt 2760
   tccccgtcaa gctctaaatc gggggctccc tttagggttc cgatttagtg ctttacggca 2820
   cctcgacccc aaaaaacttg attagggtga tggttcacgt agtgggccat cgccctgata 2880
   gacggttttt cgccctttga cgttggagtc cacgttcttt aatagtggac tcttgttcca 2940
   aactggaaca acactcaacc ctatctcggt ctattctttt gatttataag ggattttgcc 3000
   gatttcggcc tattggttaa aaaatgagct gatttaacaa aaatttaacg cgaattaatt 3060
   ctgtggaatg tgtgtcagtt agggtgtgga aagtccccag gctccccagc aggcagaagt 3120
   atgcaaagca tgcatctcaa ttagtcagca accaggtgtg gaaagtcccc aggctcccca 3180
   gcaggcagaa gtatgcaaag catgcatctc aattagtcag caaccatagt cccgccccta 3240
   actccgccca tcccgcccct aactccgccc agttccgccc attctccgcc ccatggctga 3300
   ctaatttttt ttatttatgc agaggccgag gccgcctctg cctctgagct attccagaag 3360
   tagtgaggag gcttttttgg aggcctaggc ttttgcaaaa agctcccggg agcttgtata 3420
   tccattttcg gatctgatca agagacagga tgaggatcgt ttcgcatgat tgaacaagat 3480
   ggattgcacg caggttctcc ggccgcttgg gtggagaggc tattcggcta tgactgggca 3540
   caacagacaa tcggctgctc tgatgccgcc gtgttccggc tgtcagcgca ggggcgcccg 3600
   gttctttttg tcaagaccga cctgtccggt gccctgaatg aactgcagga cgaggcagcg 3660
   cggctatcgt ggctggccac gacgggcgtt ccttgcgcag ctgtgctcga cgttgtcact 3720
   gaagcgggaa gggactggct gctattgggc gaagtgccgg ggcaggatct cctgtcatct 3780
   caccttgctc ctgccgagaa agtatccatc atggctgatg caatgcggcg gctgcatacg 3840
   cttgatccgg ctacctgccc attcgaccac caagcgaaac atcgcatcga gcgagcacgt 3900
   actcggatgg aagccggtct tgtcgatcag gatgatctgg acgaagagca tcaggggctc 3960
   gcgccagccg aactgttcgc caggctcaag gcgcgcatgc ccgacggcga ggatctcgtc 4020
   gtgacccatg gcgatgcctg cttgccgaat atcatggtgg aaaatggccg cttttctgga 4080
   ttcatcgact gtggccggct gggtgtggcg gaccgctatc aggacatagc gttggctacc 4140
   cgtgatattg ctgaagagct tggcggcgaa tgggctgacc gcttcctcgt gctttacggt 4200
   atcgccgctc ccgattcgca gcgcatcgcc ttctatcgcc ttcttgacga gttcttctga 4260
   gcgggactct ggggttcgaa atgaccgacc aagcgacgcc caacctgcca tcacgagatt 4320
   tcgattccac cgccgccttc tatgaaaggt tgggcttcgg aatcgttttc cgggacgccg 4380
   gctggatgat cctccagcgc ggggatctca tgctggagtt cttcgcccac cccaacttgt 4440
   ttattgcagc ttataatggt tacaaataaa gcaatagcat cacaaatttc acaaataaag 4500
   catttttttc actgcattct agttgtggtt tgtccaaact catcaatgta tcttatcatg 4560
   tctgtatacc gtcgacctct agctagagct tggcgtaatc atggtcatag ctgtttcctg 4620
   tgtgaaattg ttatccgctc acaattccac acaacatacg agccggaagc ataaagtgta 4680
   aagcctgggg tgcctaatga gtgagctaac tcacattaat tgcgttgcgc tcactgcccg 4740
   ctttccagtc gggaaacctg tcgtgccagc tgcattaatg aatcggccaa cgcgcgggga 4800
   gaggcggttt gcgtattggg cgctcttccg cttcctcgct cactgactcg ctgcgctcgg 4860
   tcgttcggct gcggcgagcg gtatcagctc actcaaaggc ggtaatacgg ttatccacag 4920
   aatcagggga taacgcagga aagaacatgt gagcaaaagg ccagcaaaag gccaggaacc 4980
   gtaaaaaggc cgcgttgctg gcgtttttcc ataggctccg cccccctgac gagcatcaca 5040
   aaaatcgacg ctcaagtcag aggtggcgaa acccgacagg actataaaga taccaggcgt 5100
   ttccccctgg aagctccctc gtgcgctctc ctgttccgac cctgccgctt accggatacc 5160
   tgtccgcctt tctcccttcg ggaagcgtgg cgctttctca tagctcacgc tgtaggtatc 5220
   tcagttcggt gtaggtcgtt cgctccaagc tgggctgtgt gcacgaaccc cccgttcagc 5280
   ccgaccgctg cgccttatcc ggtaactatc gtcttgagtc caacccggta agacacgact 5340
   tatcgccact ggcagcagcc actggtaaca ggattagcag agcgaggtat gtaggcggtg 5400
   ctacagagtt cttgaagtgg tggcctaact acggctacac tagaagaaca gtatttggta 5460
   tctgcgctct gctgaagcca gttaccttcg gaaaaagagt tggtagctct tgatccggca 5520
   aacaaaccac cgctggtagc ggtttttttg tttgcaagca gcagattacg cgcagaaaaa 5580
   aaggatctca agaagatcct ttgatctttt ctacggggtc tgacgctcag tggaacgaaa 5640
   actcacgtta agggattttg gtcatgagat tatcaaaaag gatcttcacc tagatccttt 5700
   taaattaaaa atgaagtttt aaatcaatct aaagtatata tgagtaaact tggtctgaca 5760
   gttaccaatg cttaatcagt gaggcaccta tctcagcgat ctgtctattt cgttcatcca 5820
   tagttgcctg actccccgtc gtgtagataa ctacgatacg ggagggctta ccatctggcc 5880
   ccagtgctgc aatgataccg cgagacccac gctcaccggc tccagattta tcagcaataa 5940
   accagccagc cggaagggcc gagcgcagaa gtggtcctgc aactttatcc gcctccatcc 6000
   agtctattaa ttgttgccgg gaagctagag taagtagttc gccagttaat agtttgcgca 6060
   acgttgttgc cattgctaca ggcatcgtgg tgtcacgctc gtcgtttggt atggcttcat 6120
   tcagctccgg ttcccaacga tcaaggcgag ttacatgatc ccccatgttg tgcaaaaaag 6180
   cggttagctc cttcggtcct ccgatcgttg tcagaagtaa gttggccgca gtgttatcac 6240
   tcatggttat ggcagcactg cataattctc ttactgtcat gccatccgta agatgctttt      6300
   ctgtgactgg tgagtactca accaagtcat tctgagaata gtgtatgcgg cgaccgagtt      6360
   gctcttgccc ggcgtcaata cgggataata ccgcgccaca tagcagaact ttaaaagtgc      6420
   tcatcattgg aaaacgttct tcggggcgaa aactctcaag gatcttaccg ctgttgagat      6480
   ccagttcgat gtaacccact cgtgcaccca actgatcttc agcatctttt actttcacca      6540
   gcgtttctgg gtgagcaaaa acaggaaggc aaaatgccgc aaaaaaggga ataagggcga      6600
   cacggaaatg ttgaatactc atactcttcc tttttcaata ttattgaagc atttatcagg      6660
   gttattgtct catgagcgga tacatatttg aatgtattta gaaaaataaa caaatagggg      6720
   ttccgcgcac atttccccga aaagtgccac ctgacgtc                               6758
<210> 5
   <211> 5845
   <212> DNA
   <213> artificial sequence
<220>
   <223> plasmide
<220>
   <221> promoter
   <222> (232)..(819)
   <223> promoteur CMV
<220>
   <221> gene
   <222> (965)..(1229)
   <223> gène d'intérêt
<220>
   <221> polyA_site
   <222> (1272)..(1496)
   <223> séquence polyA de l'HCB
<220>
   <221> gene
   <222> (2363)..(3388)
   <223> gène de résistance à l'hygromycine
<220>
   <221> gene
   <222> (4849)..(5709)
   <223> gène de résistance à l'ampicilline
<400> 5
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata      300
   tggagttccg cgttacataa cttacggtaa atggcccgcc tggctgaccg cccaacgacc      360
   cccgcccatt gacgtcaata atgacgtatg ttcccatagt aacgccaata gggactttcc      420
   attgacgtca atgggtggac tatttacggt aaactgccca cttggcagta catcaagtgt      480
   atcatatgcc aagtacgccc cctattgacg tcaatgacgg taaatggccc gcctggcatt      540
   atgcccagta catgacctta tgggactttc ctacttggca gtacatctac gtattagtca      600
   tcgctattac catggtgatg cggttttggc agtacatcaa tgggcgtgga tagcggtttg      660
   actcacgggg atttccaagt ctccacccca ttgacgtcaa tgggagtttg ttttggcacc      720
   aaaatcaacg ggactttcca aaatgtcgta acaactccgc cccattgacg caaatgggcg      780
   gtaggcgtgt acggtgggag gtctatataa gcagagctct ctggctaact agagaaccca      840
   ctgcttactg gcttatcgaa attaatacga ctcactatag ggagacccaa gctggctagc      900
   gtttaaactt aagcttggta ccgagctcgg atccactagt ccagtgtggt ggaattctgc      960
   agatatcatg attccagcag tggtcttgct cttactcctt ttggttgaac aagcagcggc     1020
   cctgggagag cctcagctct gctatatcct ggatgccatc ctgtttctgt atggaattgt     1080
   cctcaccctc ctctactgtc gactgaagat ccaagtgcga aaggcagcta taaccagcta     1140
   tgagaaatca gatggtgttt acacgggcct gagcaccagg aaccaggaga cttacgagac     1200
   tctgaagcat gagaaaccac cacagtagct cgagtctaga gggcccgttt aaacccgctg     1260
   atcagcctcg actgtgcctt ctagttgcca gccatctgtt gtttgcccct cccccgtgcc     1320
   ttccttgacc ctggaaggtg ccactcccac tgtcctttcc taataaaatg aggaaattgc     1380
   atcgcattgt ctgagtaggt gtcattctat tctggggggt ggggtggggc aggacagcaa     1440
   gggggaggat tgggaagaca atagcaggca tgctggggat gcggtgggct ctatggcttc     1500
   tgaggcggaa agaaccagct ggggctctag ggggtatccc cacgcgccct gtagcggcgc     1560
   attaagcgcg gcgggtgtgg tggttacgcg cagcgtgacc gctacacttg ccagcgccct     1620
   agcgcccgct cctttcgctt tcttcccttc ctttctcgcc acgttcgccg gctttccccg     1680
   tcaagctcta aatcggggca tccctttagg gttccgattt agtgctttac ggcacctcga     1740
   ccccaaaaaa cttgattagg gtgatggttc acgtagtggg ccatcgccct gatagacggt     1800
   ttttcgccct ttgacgttgg agtccacgtt ctttaatagt ggactcttgt tccaaactgg     1860
   aacaacactc aaccctatct cggtctattc ttttgattta taagggattt tggggatttc     1920
   ggcctattgg ttaaaaaatg agctgattta acaaaaattt aacgcgaatt aattctgtgg     1980
   aatgtgtgtc agttagggtg tggaaagtcc ccaggctccc caggcaggca gaagtatgca     2040
   aagcatgcat ctcaattagt cagcaaccag gtgtggaaag tccccaggct ccccagcagg     2100
   cagaagtatg caaagcatgc atctcaatta gtcagcaacc atagtcccgc ccctaactcc     2160
   gcccatcccg cccctaactc cgcccagttc cgcccattct ccgccccatg gctgactaat 2220
   tttttttatt tatgcagagg ccgaggccgc ctctgcctct gagctattcc agaagtagtg 2280
   aggaggcttt tttggaggcc taggcttttg caaaaagctc ccgggagctt gtatatccat 2340
   tttcggatct gatcagcacg tgatgaaaaa gcctgaactc accgcgacgt ctgtcgagaa 2400
   gtttctgatc gaaaagttcg acagcgtctc cgacctgatg cagctctcgg agggcgaaga 2460
   atctcgtgct ttcagcttcg atgtaggagg gcgtggatat gtcctgcggg taaatagctg 2520
   cgccgatggt ttctacaaag atcgttatgt ttatcggcac tttgcatcgg ccgcgctccc 2580
   gattccggaa gtgcttgaca ttggggaatt cagcgagagc ctgacctatt gcatctcccg 2640
   ccgtgcacag ggtgtcacgt tgcaagacct gcctgaaacc gaactgcccg ctgttctgca 2700
   gccggtcgcg gaggccatgg atgcgatcgc tgcggccgat cttagccaga cgagcgggtt 2760
   cggcccattc ggaccgcaag gaatcggtca atacactaca tggcgtgatt tcatatgcgc 2820
   gattgctgat ccccatgtgt atcactggca aactgtgatg gacgacaccg tcagtgcgtc 2880
   cgtcgcgcag gctctcgatg agctgatgct ttgggccgag gactgccccg aagtccggca 2940
   cctcgtgcac gcggatttcg gctccaacaa tgtcctgacg gacaatggcc gcataacagc 3000
   ggtcattgac tggagcgagg cgatgttcgg ggattcccaa tacgaggtcg ccaacatctt 3060
   cttctggagg ccgtggttgg cttgtatgga gcagcagacg cgctacttcg agcggaggca 3120
   tccggagctt gcaggatcgc cgcggctccg ggcgtatatg ctccgcattg gtcttgacca 3180
   actctatcag agcttggttg acggcaattt cgatgatgca gcttgggcgc agggtcgatg 3240
   cgacgcaatc gtccgatccg gagccgggac tgtcgggcgt acacaaatcg cccgcagaag 3300
   cgcggccgtc tggaccgatg gctgtgtaga agtactcgcc gatagtggaa accgacgccc 3360
   cagcactcgt ccgagggcaa aggaatagca cgtgctacga gatttcgatt ccaccgccgc 3420
   cttctatgaa aggttgggct tcggaatcgt tttccgggac gccggctgga tgatcctcca 3480
   gcgcggggat ctcatgctgg agttcttcgc ccaccccaac ttgtttattg cagcttataa 3540
   tggttacaaa taaagcaata gcatcacaaa tttcacaaat aaagcatttt tttcactgca 3600
   ttctagttgt ggtttgtcca aactcatcaa tgtatcttat catgtctgta taccgtcgac 3660
   ctctagctag agcttggcgt aatcatggtc atagctgttt cctgtgtgaa attgttatcc 3720
   gctcacaatt ccacacaaca tacgagccgg aagcataaag tgtaaagcct ggggtgccta 3780
   atgagtgagc taactcacat taattgcgtt gcgctcactg cccgctttcc agtcgggaaa 3840
   cctgtcgtgc cagctgcatt aatgaatcgg ccaacgcgcg gggagaggcg gtttgcgtat 3900
   tgggcgctct tccgcttcct cgctcactga ctcgctgcgc tcggtcgttc ggctgcggcg 3960
   agcggtatca gctcactcaa aggcggtaat acggttatcc acagaatcag gggataacgc 4020
   aggaaagaac atgtgagcaa aaggccagca aaaggccagg aaccgtaaaa aggccgcgtt 4080
   gctggcgttt ttccataggc tccgcccccc tgacgagcat cacaaaaatc gacgctcaag 4140
   tcagaggtgg cgaaacccga caggactata aagataccag gcgtttcccc ctggaagctc 4200
   cctcgtgcgc tctcctgttc cgaccctgcc gcttaccgga tacctgtccg cctttctccc 4260
   ttcgggaagc gtggcgcttt ctcaatgctc acgctgtagg tatctcagtt cggtgtaggt 4320
   cgttcgctcc aagctgggct gtgtgcacga accccccgtt cagcccgacc gctgcgcctt 4380
   atccggtaac tatcgtcttg agtccaaccc ggtaagacac gacttatcgc cactggcagc 4440
   agccactggt aacaggatta gcagagcgag gtatgtaggc ggtgctacag agttcttgaa 4500
   gtggtggcct aactacggct acactagaag gacagtattt ggtatctgcg ctctgctgaa 4560
   gccagttacc ttcggaaaaa gagttggtag ctcttgatcc ggcaaacaaa ccaccgctgg 4620
   tagcggtggt ttttttgttt gcaagcagca gattacgcgc agaaaaaaag gatctcaaga 4680
   agatcctttg atcttttcta cggggtctga cgctcagtgg aacgaaaact cacgttaagg 4740
   gattttggtc atgagattat caaaaaggat cttcacctag atccttttaa attaaaaatg 4800
   aagttttaaa tcaatctaaa gtatatatga gtaaacttgg tctgacagtt accaatgctt 4860
   aatcagtgag gcacctatct cagcgatctg tctatttcgt tcatccatag ttgcctgact 4920
   ccccgtcgtg tagataacta cgatacggga gggcttacca tctggcccca gtgctgcaat 4980
   gataccgcga gacccacgct caccggctcc agatttatca gcaataaacc agccagccgg 5040
   aagggccgag cgcagaagtg gtcctgcaac tttatccgcc tccatccagt ctattaattg 5100
   ttgccgggaa gctagagtaa gtagttcgcc agttaatagt ttgcgcaacg ttgttgccat 5160
   tgctacaggc atcgtggtgt cacgctcgtc gtttggtatg gcttcattca gctccggttc 5220
   ccaacgatca aggcgagtta catgatcccc catgttgtgc aaaaaagcgg ttagctcctt 5280
   cggtcctccg atcgttgtca gaagtaagtt ggccgcagtg ttatcactca tggttatggc 5340
   agcactgcat aattctctta ctgtcatgcc atccgtaaga tgcttttctg tgactggtga 5400
   gtactcaacc aagtcattct gagaatagtg tatgcggcga ccgagttgct cttgcccggc 5460
   gtcaatacgg gataataccg cgccacatag cagaacttta aaagtgctca tcattggaaa 5520
   acgttcttcg gggcgaaaac tctcaaggat cttaccgctg ttgagatcca gttcgatgta 5580
   acccactcgt gcacccaact gatcttcagc atcttttact ttcaccagcg tttctgggtg 5640
   agcaaaaaca ggaaggcaaa atgccgcaaa aaagggaata agggcgacac ggaaatgttg 5700
   aatactcata ctcttccttt ttcaatatta ttgaagcatt tatcagggtt attgtctcat 5760
   gagcggatac atatttgaat gtatttagaa aaataaacaa ataggggttc cgcgcacatt 5820
   tccccgaaaa gtgccacctg acgtc 5845